# EUROPÄISCHE PATENTSCHRIFT

(11) **EP 1 274 511 B1**
(45) Veröffentlichungstag und Bekanntmachung des Hinweises auf die Patenterteilung: **18.08.2004**
(21) Anmeldenummer: 01929522.9
(22) Anmeldetag: 06.04.2001
(51) Int. Cl.: B01L 3/02, B01J 4/02, G01F 11/00, G01N 27/447, B01L 3/00

(54) **VERFAHREN UND VORRICHTUNG ZUR MIKRODOSIERUNG KLEINSTER FLÜSSIGKEITSMENGEN FÜR BIOPOLYMERARRAYS**
METHOD AND DEVICE FOR MICRODOSING THE SMALLEST AMOUNTS OF LIQUID FOR BIOPOLYMER ARRAYS
PROCEDE ET DISPOSITIF DE MICRODOSAGE D'INFIMES QUANTITES DE LIQUIDE POUR JEUX ORDONNES D'ECHANTILLONS BIOPOLYMERES

(30) Priorität: 10.04.2000 DE 10017791
(43) Veröffentlichungstag der Anmeldung: 15.01.2003
(73) Patentinhaber: BASF AKTIENGESELLSCHAFT, 67056 Ludwigshafen (DE); DEUTSCHES KREBSFORSCHUNGSZENTRUM STIFTUNG DES ÖFFENTLICHEN RECHTS, 69120 Heidelberg (DE)
(72) Erfinder: EIPEL, Heinz, 64625 Bensheim (DE); BEIER, Markus, 69121 Heidelberg (DE); MATYSIAK, Stefan, 88069 Tettnang (DE)
(74) Vertreter: Isenbruck, Günter, Dr.
(86) Internationale Anmeldenummer: PCT/EP2001/004000
(87) Internationale Veröffentlichungsnummer: WO 2001/076745

(56) Entgegenhaltungen:
- WO-A-98/29736
- US-A- 4 908 112
- US-A- 5 384 093
- US-A- 5 900 130
- US-A- 6 001 229

## Beschreibung

Die Erfindung bezieht sich auf ein Verfahren und eine Vorrichtung zur Mikrodosierung kleinster Flüssigkeitsmengen für Biopolymerarrays oder Biopolymerfelder.

Zur hochparallelen Analytik von Biopolymeren wie beispielsweise Nucleinsäuren, Proteinen oder Polysacchariden werden Mikropolymerfelder, auch als Mikroarrays bezeichnet, eingesetzt. Zur Herstellung solcher Felder oder Arrays müssen sehr kleine in Flüssigkeiten gelöste oder suspendierte Biopolymerproben im Bereich von Picolitern bis Nanolitern in regelmäßigen Anordnungen auf Substratoberflächen beispielsweise auf Objektträger aufgebracht werden. Herkömmliche Pipettierverfahren versagen bei solchen kleinen Flüssigkeitsmengen.

Da eine genaue Dosierung der zu übertragenden Menge bisher sehr schwierig ist, wird meist auf eine genaue Dosierung verzichtet, und die Mengen werden durch eine mechanisch kontaktierende Anordnung, ähnliche einer Schreibfeder, übertragen. Solche eingesetzten Schreibfedern haben jedoch nur eine begrenzte Flüssigkeitsaufnahmekapazität, so daß das Beschicken einer Vielzahl von Substratträgeroberflächen mit einer Schreibfederfüllung nicht möglich ist. Zur Kapazitätserweiterung der eingesetzten Schreibfedern hat man versucht, an diesen Einkerbungen oder Furchen vorzusehen, um eine größere Menge von zu beschickendem Probensubstrat an der Schreibfeder aufzunehmen. Damit ließ sich zwar die Kapazität der Schreibfedern erhöhen, so daß mit einer Schreibfederfüllung eine größere Anzahl von Biopolymerflecken auf einen Objektträger aufgebracht werden konnte, jedoch gestaltete sich die Reinigung einer solcherart beschaffenen, mit Furchen und Schlitzen versehenen Schreibfeder sehr schwierig. Es muß dafür Sorge getragen werden, daß auch die die Kapazität der Schreibfeder erweiternden Furchen und Schlitze von Resten aus vorhergehenden Proben Beschickungsläufe gereinigt werden, wenn mit der Schreibfeder eine neue Biopolymerprobe auf einen zu beschickenden Objektträger aufgebracht werden soll.

Um die Meßfehler bei der Analytik mit Hilfe solcher Biopolymerarrays ausreichend klein zu halten, werden üblicherweise interne Standards verwendet

Die Schrift WO-A-98 29 736 offenbart ein Verfahren zur Herstellung von Biopolymerarrays durch Mikrodosierung kleinster Flüssigkeitsmengen, bei dem Probenflüssigkeit am selben Ende einer z.B. als Kapillare ausgebildeten Zuführeinrichtung in einem Zug eingesaugt und zur Erzeugung mehrerer "Array Elemente" absatzweise wieder abgegeben wird. Die Biopolymerarrays werden dabei bevorzugt dadurch erzeugt, dass die Kapillaren eines Kapillarbündels mit ihrem einen Ende in Biopolymer enthaltende Probengefäße eintauchen und, durch elektro-osmotische Kräfte unterstützt, über ihr anderes Ende, Biopolymerproben dosiert auf eine Detektionsoberfläche abgeben.

Angesichts der aus dem Stand der Technik bekannten Lösungen und der diesen anhaftenden Nachteile liegt der Erfindung die Aufgabe zugrunde, Biopolymerarrays auf eine einfache und zuverlässig erfolgende Weise mit geringsten Flüssigkeitsmengen zu beschicken.

Gemäß Anspruch 1 wird dieses Aufgabe gelöst, in dem bei einem Verfahren zur Mikrodosierung kleinster Flüssigkeitsmengen für die Herstellung von Biopolymerarrays, wobei die zu analysierenden Probenflüssigkeit mittels einer Zuführeinrichtung zuführbar sind, die mit einem Spülfluid verbindbar ist, an die Zuführeinrichtung eine umpolbare elektrische Spannung anlegbar ist, so daß der sich einstellende elektro-osmotische Fluß zum Transport der Probenflüssigkeit auf eine Detektionsoberfläche nutzbar ist.

Die mit dem erfindungsgemäß vorgeschlagenen Verfahren erzielbaren Vorteile sind vor allem daran zu erblicken, daß mittels einer an die Pipettierspitze des das Probensubstrat aufnehmenden Kapillarröhrchens anlegbaren Spannung eine genaueste Dosierung kleinster Flüssigkeitsmengen zu dem Zeitpunkt erfolgen kann, an dem die Pipettierspitze an die Detektionsfläche des jeweiligen Objektträgers angestellt ist. Werden mehrere parallel zueinander betriebene Pipettierspitzen von Kapillarröhren durch Anlegen der den Transport der Probenflüssigkeit erzeugenden Spannung verwendet, so lassen sich in präziser Weise unter Erzielung hochgenauer Abstände voneinander, einzelne Biopolymerflecken auf den Detektionsoberflächen von Objektträgern kostengünstig und schnell anordnen.

Bei dem erfindungsgemäß vorgeschlagenen Verfahren wird durch Anlegen einer elektrischen Spannung an eine Antriebskapillare und der Zuführeinrichtung das Ansaugen eines Biopolymers aus einem Probenvorrat und nach Umpolen der Spannung die Abgabe der zu dosierenden Flüssigkeit bewirkt. Zur Abgabe der Probenflüssigkeitsmengen und zur Erzeugung der Biopolymerflecken auf der Oberfläche des Detektionsfeldes bedarf es demnach keiner mechanisch zu betätigenden Komponenten innerhalb des Hohlraumes des Kapillarröhrchens mehr..

Gemäß einer weiteren vorteilhaften Ausgestaltung des erfindungsgemäß vorgeschlagenen Verfahrens wird die elektrische Spannung zum Transport des Probensubstrates zwischen Kapillarkopf und Kapillarraum der Antriebskapillare angelegt. Dies gestattet eine Zuführung der elektrischen Zuleitung im oberen Teil der Glaskapillare, an dem der Pipettierspitze gegenüberliegenden Ende der Glaskapillare.

Gemäß eines weiteren vorteilhaften Gedankens der erfindungsgemäßen Lösung ist die Pipettierspitze des Kapillarraumes in drei Dimensionen verfahrbar. Neben einer Verfahrbarkeit der Pipettierspitze in X bzw. Y-Richtung oberhalb des Detektionsfeldes läßt sich die Pipettierspitze in Z-Richtung an die Oberfläche des Detektionsfeldes anstellen, bevor eine einen Flüssigkeitsausstoß bewirkende Spannung an dem Inhalt des Kapillarhohlraums angelegt wird.

Zur Vermeidung von Probenflüssigkeitsverlusten und Störungen im Ausbringen des Biopolymermusters auf der Detektionsoberfläche, erfolgt eine Umpolung der elektrischen Spannung, die einen Austritt des Probensubstrates aus dem Kapillarraum der Kapillarröhre bewirkt, in der an die Detektionsoberfläche angestellten Position der Pipettierspitze.

Schließlich ist gemäß des erfindungsgemäß vorgeschlagenen Verfahrens zur Dosierung kleinster Flüssigkeitsmengen vorgesehen, die Antriebskapillare und deren Pipettierspitze mittels eines Ventiles mit einer Pufferlösung in Verbindung zu bringen, die in einem druckbeaufschlagten Behälter bevorratet wird, wobei als Pufferlösung der Erzeugung eines elektro-osmotischen Druckes günstige Pufferlösung mit entsprechendem pH-Wert und Ionenkonzentration Verwendung findet.

Schließlich ist vorgesehen, über eine auf der Oberfläche des Objektträgers, d. h. auf die Detektionsoberfläche aufzubringende leitfähige Schicht eine elektrophoretische Abscheidung geladener Biopolymerspezies auf dem Objektträger vorzunehmen. Mit dieser Variante des erfindungsgemäß vorgeschlagenen Verfahrens können Analyseschritte nachfolgender Analyseoperationen bereits beim Aufbringen und Erzeugen der Biopolymerarrays vorgenommen werden.

Gemäß der in Anspruch 6 weiter vorgeschlagenen Vorrichtung zur Mikrodosierung kleinster Flüssigkeiten, sind in die elektrische Zuleitung zum Anlegen einer Spannung an einer Antriebskapillare die Spannung umpolende Schaltelemente integriert, die über einen elektrischen Kontakt mit dem Inhalt eines Pufferbehälters in Verbindung stehen. Mit der erfindungsgemäß vorgeschlagenen Vorrichtung kann durch einfaches Umpolen der Spannung aufgrund des elektro-osmotischen Flusses im Probensubstrat ein Ausbringen kleinster Flüssigkeitsmengen durch die Pipettierspitze im abgesenkten Zustand oberhalb eines Detektionsfeldes eines Objektträgers erfolgen.

Zur Sicherstellung einer kontinuierlichen Versorgung der Antriebskapillare mit Pufferlösung, ist an einem hinter einem Ventil befindlichen Abzweig der Antriebskapillare ein Strömungswiderstand oberhalb eines Pufferlösungsbehälters aufgenommen. Durch geeignete Dimensionierung des Strömungswiderstandes ist eine blasen- und hohlraumfreie Versorgung der Antriebskapillare mit Pufferfluid erzielbar.

In vorteilhafter Weise läßt sich die Pipettierspitze einer Antriebskapillare oder die Pipettierspitzen mehrerer Antriebskapillaren über eine einfache gestaltete X-, Y-Positioniereinheit oberhalb des Detektionsfeldes verfahren und es lassen sich dadurch die richtigen Positionen einstellen, in denen die Biopolymerflecken auf die Detektionsfläche aufzubringen sind. Neben der Verfahrbarkeit der Pipettierspitze in X- bzw. Y-Richtung kann über die Positioniereinheit - beispielsweise ein handelsüblicher Plotter - auch ein Anstellen der Pipettierspitze in Z-Richtung an die Oberfläche des Detektionsfeldes erfolgen.

Vorteilhafterweise ist die Antriebskapillare bzw. die Pipettierspitze aus Glas oder Quarz gefertigt.

Die Pipettierspitze der Mikrokapillare ist in vorteilhafter Weise mit einer auf einen kleinen Durchmesser ausgezogenen Spitze mit einem Spitzendurchmesser im Bereich zwischen 10 µm und 1000 µm ausgezogen. Besonders bevorzugt ist der Durchmesserbereich der Pipettierspitze zwischen 50 µ m und 300 µm.

Zur Sicherstellung einer Masseverbindung ist zwischen Pipettierspitze und Antriebskapillare eine elektrische Erdung vorgesehen.

Schließlich ist zwischen Pipettierspitze und Antriebskapillare eine Verbindung vorgesehen zur Erzeugung eines elektro-osmotischen Flusses, wobei eine Platindraht-Elektrode im Kapillarkopf der Antriebskapillare aufgenommen ist, sowie der weitere elektrische Anschluß des Endes der Antriebskapillare in ein elektrisch kontaktiertes Puffergefäß eintaucht. Somit ist der Spannungskreis an der Antriebskapillare lediglich durch den Umpolschalter unterbrochen und kann durch diesen in einer entsprechend der geforderten Beschickungsfrequenz der Biopolymerflecken auf die Detektionsfläche unterbrochen bzw. geschlossen werden.

Anhand der Zeichnung wird die Erfindung nachfolgend detailliert erläutert.

In der einzigen Figur ist der schematisch wiedergegebene Aufbau einer erfindungsgemäß vorgeschlagenen Vorrichtung zur Aufbringung kleinster Flüssigkeitsmengen im Pico- bzw. Nanoliterbereich dargestellt.

Als Pipettierspitze 1 zum Aufbringen einer Probenflüssigkeit auf die Detektionsfläche 18 eines Objektträgers 9 wird eine sehr kostengünstig herstellbare Glaskapillare mit einer auf einen Durchmesser von beispielsweise 200 µm ausgezogenen Spitze verwendet. Diese Kapillare ist an ihrem Ende über einen Mikroschlauch an eine Antriebskapillare 2 aus Glas oder Quarz, wie sie in der Gaschromatographie üblich ist, angeschlossen. In den Schlauchanschluß des Mikroschlauches ist eine Platindrahtelektrode 3 zur Herstellung eines elektrischen Kontaktes eingesteckt. Am gegenüberliegenden Ende der Antriebskapillare 2 befindet sich ein zweiter elektrischer Anschluß 4, der in den in einem Pufferbehälter 14 aufgenommenen Inhalt von Pufferlösung hineinragt. Das in dem Pufferbehälter 14 aufgenommene Fluid wird durch einen Leitungsabzweig 16, in dem ein Strömungswiderstand 13 aufgenommen ist, kontinuierlich versorgt, so daß der elektrische Anschluß 4 stets mit dem Fluid in der Antriebskapillare 2 in Verbindung steht.

Zu Beginn eines Pipettiervorganges wird zunächst die Pipettierspitze 1 einer Glaskapillare mit einer X-Y-Positioniervorrichtung beispielsweise in Gestalt eines handelsüblichen graphischen Plotters oder einer anderen X-Y-Positioniereinrichtung über einen Abfallbehälter 7 gefahren. Anschließend wird ein vor der Antriebskapillare 2 angeordnetes Ventil 5 kurzzeitig geöffnet und so die Antriebskapillare 2 samt Pipettierspitze 1, positioniert über dem Abfallbehälter 7, durchgehend mit frischer Pufferlösung, deren pH- und Ionenkonzentration zur Erzeugung eines elektro-osmotischen Flusses in der Antriebskapillare 2 geeignet eingestellt ist, aus der über einen Gasanschluß 6 unter Druck stehender Vorratsbehälter 11 gefüllt und somit gleichzeitig die Pipettierspitze 1 über dem Abfallbehälter 7 ausgeblasen. Der im erwähnten Abzweig 16 befindliche Strömungswiderstand 13, beispielsweise in Form einer Fritte bewirkt, daß eine kleine Menge Pufferfluid in den Pufferbehälter 14 gedrückt wird, so daß sichergestellt ist, daß die Antriebskapillare 2, die in die Pipettierspitze mündet, stets mit einem durchgehend und kontinuierlich sich erstreckenden Puffervorrat beaufschlagt ist.

Zwischen der Zuleitung 3 und dem elektrischen Kontakt 4 zum Pufferbehälter 14 ist ein Schaltelement 10, hier in schematischer Darstellung wiedergegeben, aufgenommen. An die Schaltkontakte des Schaltelementes 10 schließen sich zwei elektrische Spannungsquellen, mit Bezugszeichen 12 a bzw. 12 b bezeichnet, an, die über eine Erdung 17 mit Masse verbunden sind.

Zum Ansaugen der im Biopolymergefäß 8 bereitgestellten zu pipettierenden Biopolymerlösung wird über den Schalter 10 an die beiden elektrischen Anschlüsse 3 bzw. 4 eine geeignet gepolte elektrische Spannung zur Erzeugung eines rückwärts gerichteten elektro-osmotischen Flusses in der Antriebskapillare 2 angelegt. Zu diesem Zeitpunkt ist die Pipettierspitze 1 in Z-Richtung gesehen in das Probengefäß 8 eingetaucht, so daß entsprechend der angelegten Spannung durch die Öffnung der Pipettierspitze 1 Probensubstrat angesaugt werden kann. Ist genügend Pipettiergut aus dem Biopolymergefäß 8, hier beispielsweise ein Töpfchen einer Mikrotiterplatte angesaugt, positioniert der Mikro-Pipettierautomat, d.h. die X-Y-Positioniervorrichtung die Pipettierspitze 1 über das zu beschickende Substrat. Das Substrat kann beispielsweise ein Objektträger 9 sein, wie sie in der Mikroskopie häufig verwendet werden. Auf dem Objektträger 9 ist eine Objektträgeroberfläche 18 vorgesehen, auf die die einzelnen aus der Pipettierspitze 1 austretenden Biopolymertröpfchen aufgebracht werden. Die Detektionsfläche 18 kann auch eine das Biopolymer chemisch bindende oder physiko-chemisch wechselwirkende Oberfläche sein. Mittels der X-Y-Zufuhrvorrichtung, welche zudem eine Absenkung der Pipettierspitze 1 in Richtung auf das Detektionsfeld 18 ermöglicht, erfolgt der Auftrag der Biopolymerflecken auf die Objektträgeroberfläche 18. Zu diesem Zweck wird über den Schalter 10 eine umgepolte elektrische Spannung für eine vorwählbare Zeit an die Antriebskapillare 2 angelegt, wodurch die zu pipettierende Flüssigkeit aus der Pipettierspitze 1 durch den nun in umgekehrter Richtung laufenden elektro-osmotischen Fluß herausgedrückt wird und auf die Detektionsoberfläche 18 des Objektträgers 9 austritt. Dadurch läßt sich die Probenflüssigkeit sowohl auf die Detektionsoberfläche 18 als auch in ein anderes Gefäß ausbringen. Alternativ zur Verwendung zweier Spannungsquellen kann auch eine einzige Spannungsquelle mit einem entsprechenden Umschaltelement verwendet werden, auch sind andere Varianten beispielweise der Massenverbindung durchaus möglich.

Durch entsprechende Einstellung der den elektro-osmotischen Fluß beeinflussenden Parameter, wie hauptsächlich der Ionenkonzentration und des pH-Wertes des Puffers sowie der Höhe der angelegten elektrischen Spannung, kann die Menge der abgegebenen Flüssigkeitsmenge dosiert bzw. bei der Erzeugung der einzelnen Biopolymerflecken auf der Detektionsoberfläche 18 des Objektträgers annähernd konstant gehalten werden. Dadurch läßt sich ein Biopolymermuster 19 auf der Detektionsoberfläche 18 des Objektträgers 9 erzeugen, welches Biopolymerflecken enthält, die in regelmäßigen Abständen 20 voneinander sowohl in X- als auch in Y-Richtung angeordnet sind.

Zur Beschleunigung und zur elektrochemischen Aktivierung der Anbindung der Biopolymerflecken an eine geeignete, mit dem Biopolymer chemisch oder physiko-chemisch wechselwirkende Oberfläche 18 des Objektträgers 9, kann nach Kontaktierung der Pipettierspitze 1 zusätzlich eine geeignet gepolte elektrische Spannung zwischen dem Anschluß 3 der Pipettierspitze 1 und einer elektrisch leitenden Oberfläche auf dem Objektträger 9 angelegt werden. Dadurch kann eine elektrophoretische Abscheidung elektrisch geladener Biopolymerspezies bereits auf dem Objektträger kurz nach deren Aufbringen erfolgen, was für eine weitere Analyse und Probenauswertung sehr förderlich ist.

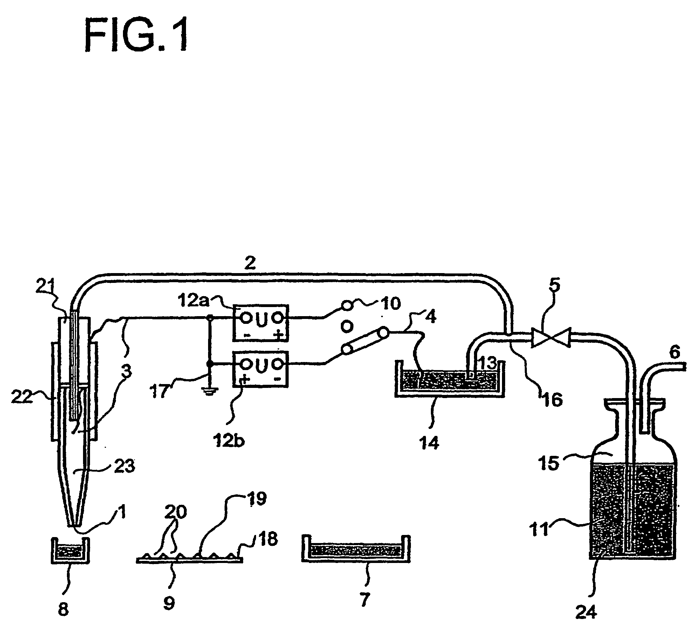

Wie Fig. 1 entnommen werden kann, ist der Kopf der Antriebskapillare 2 in einem Kapillarkopf 21 aufgenommen, der seinerseits von einer Fassung 22, beispielsweise ein kurzes Schlauchstück, umschlossen ist. In die Fassung 22 ist in geeigneter Weise die gläserne oder aus Quarz beschaffene Pipettierspitze 1 eingelassen, die einen Hohlraum 23 aufweist, in den die zu pipettierende Probenflüssigkeit angesaugt bzw. bei Umkehrung des elektro-osmotischen Flusses aus dem Hohlraum 23 ausgestoßen wird. Die Pipettierspitze 1, die vorzugsweise aus Glas beschaffen ist, kann Öffnungen im Bereich zwischen 10 µm und 1000 µm enthalten, wobei vorzugsweise an der Pipettierspitzenöffnung 1 ein Durchmesser zwischen 50 µm und 300 µm ausgebildet ist.

### Bezugszeichenliste

- 1: Pipettierspitze
- 2: Antriebskapillare
- 3: elektrischer Kontakt
- 4: elektrischer Anschluß
- 5: Ventil
- 6: Gasanschluß
- 7: Abfallbehälter
- 8: Biopolymergefäß
- 9: Objektträger
- 10: Umpolschalter
- 11: Vorratsflasche
- 12a: elektrische Spannungsquelle +
- 12b: elektrische Spannungsquelle -
- 13: Strömungswiderstand
- 14: Pufferbehälter
- 15: Druckleitung
- 16: T-Stück
- 17: Masseanschluß
- 18: Objektträgeroberfläche
- 19: Biopolymermuster
- 20: Abstand der Biopolymerflecke
- 21: Kapillarkopf
- 22: Fassung
- 23: Kapillarhohlraum
- 24: Pufferlösung

## Patentansprüche

1. Verfahren zur Herstellung von Biopolymerarrays durch Mikrodosierung kleinster Flüssigkeitsmengen, bei dem zunächst eine Zuführeinrichtung, eine die Zuführeinrichtung mit einem Puffergefäß (14) verbindende Antriebskapillare (2) und das Puffergefäß aus einem an die Antriebskapillare anschließbaren Vorratsbehälter (11) mit einer Pufferlösung (24) gefüllt werden, die die Erzeugung eines elektro-osmotischen Flusses ermöglicht bzw. gegebenenfalls als Spülfluid dient, anschließend durch Anlegen einer elektrischen Spannung an die . Antriebskapillare (2) ein elektro-osmotischer Fluss erzeugt und Biopolymer in die mit einem Ende in ein Gefäß (8) eingetauchte Zuführeinrichtung eingesaugt wird und schließlich nach Umpolen der Spannung Biopolymerproben dosiert auf eine Detektionsoberfläche (18) abgegeben werden.

2. Verfahren nach Anspruch 1, **dadurch gekennzeichnet, daß** die Zuführeinrichtung einen Kapillarraum (23) mit einer Pipettierspitze (1) umfaßt, wobei die Pipettierspitze (1) des Kapillarraumes (23) dreidimensional verfahrbar ist.

3. Verfahren nach Anspruch 2, **dadurch gekennzeichnet, daß** die Umpolung der elektrischen Spannung, die einen Austritt von Probenflüssigkeit aus dem in der Zuführeinrichtung enthaltenen Kapillarraum (23) bewirkt, nach Erreichen der an eine Detektionsoberfläche (18) angestellten Position der von der Zuführeinrichtung umfassten Pipettierspitze (1) erfolgt.

4. Verfahren nach einem der Ansprüche 2 oder 3, **dadurch gekennzeichnet, daß** die die Antriebskapillare (2) und die Pipettierspitze (1) durch Öffnen eines Ventiles (5) mit der Pufferlösung (24) versorgbar sind, die druckbeaufschlagt in dem Vorratsbehälter bevorratet wird und einen für die Erzeugung eines elektro-osmotischen Druckes geeigneten pH-Wert, sowie eine geeignete Ionenkonzentration aufweist.

5. Verfahren nach einem der Ansprüche 1 bis 4, **dadurch gekennzeichnet, daß** durch eine elektrisch leitfähige Schicht auf einer Objektträgeroberfläche (18) eine elektrophoretische Abscheidung geladener Biopolymerspezies auf der Objektträgeroberfläche (18) vorgenommen wird.

6. Vorrichtung zur Herstellung von Biopolymerarrays durch Mikrodosierung kleinster Flüssigkeitsmengen mit einer Zuführeinrichtung zum Zuführen von zu analysierenden Probensubstraten einem Puffergefäß (14), einer Antriebskapillare (2), die Zuführeinrichtung und Puffergefäß (14) verbindet und mit Verbindungsleitungen zum Verbinden der Antriebskapillare (2) mit einem ein Spülfluid (24) enthaltenden Vorratsbehälter, wobei zum Anlegen einer Spannung zwischen der Zuführeinrichtung und dem Puffergefäß (14) an die Antriebskapillare eine elektrische Zuleitung (3) und an dem Puffergefäß (14) ein elektrischer Kontakt (4) und an die elektrische Zuleitung (3) umpolbare Schaltelemente (10) und elektrische Spannungsquellen (12a, 12b) angeschlossen sind.

7. Vorrichtung nach Anspruch 6, **dadurch gekennzeichnet, daß** in einen Abzweig (16) der Antriebskapillare (2) ein Strömungswiderstand (13) eingebracht ist.

8. Vorrichtung nach einem der Ansprüche 6 oder 7, **dadurch gekennzeichnet, daß** die Zuführeinrichtung eine Pipettierspitze (1) umfaßt, zu deren Positionierung in Bezug auf ein Detektionsfeld (18) eine X-, Y-Positioniervorrichtung vorgesehen ist, die ein Anstellen der Pipettierspitze (1) an die Oberfläche eines Objektträgers (9) bewirkt.

9. Vorrichtung nach einem der Ansprüche 6 bis 8, **dadurch gekennzeichnet, daß** die Antriebskapillare (2) aus Glas oder Quarz besteht.

10. Vorrichtung nach einem der Ansprüche 8 oder 9 in Verbindung mit 8, **dadurch gekennzeichnet, daß** die Pipettierspitze (1) eine aus einer Glaskapillare auf einen kleinen Durchmesser ausgezogenen Spitze ist, wobei die Spitze einen Durchmesser im Bereich zwischen 10 µm und 1000 µm besitzt.

11. Vorrichtung nach Anspruch 10, **dadurch gekennzeichnet, daß** die Pipettierspitze (1) eine aus einer Glaskapillare auf einen kleinen Durchmesser ausgezogenen Spitze mit einem Durchmesser im Bereich zwischen 50 µm und 300 µm ist.

12. Vorrichtung nach einem der Ansprüche 8, 9 in Verbindung mit 8, 10 und 11, **dadurch gekennzeichnet, daß** eine elektrische Erdung zwischen Pipettierspitze (1) und Antriebskapillare (2) vorgesehen ist.

13. Vorrichtung nach einem der Ansprüche 8 bis 12, **dadurch gekennzeichnet, daß** eine Platindrahtelektrode (3) in einem in der Zuführeinrichtung enthaltenen Kapillarkopf (21) vorgesehen ist.

## Claims

1. A process for the production of biopolymer arrays by micrometering extremely small quantities of liquid, which comprises initially a supply device, a drive capillary (2) which connects the supply device with a buffer vessel (14) and the buffer vessel being filled, from a stock container (11) connectable to the drive capillary, with a buffer solution (24) which enables the generation of an electro-osmotic flow and, if appropriate, serves as a rinsing fluid, then generating an electro-osmotic flow by applying an electric voltage to the drive capillary (2) and sucking biopolymer into the supply device which dips with one end into a vessel (8), and finally, after reversal of the voltage, biopolymer samples being released onto a detection surface (18) in a metered fashion.

2. A process as claimed in claim 1, wherein the supply device comprises a capillary space (23) having a pipetting tip (1), and the pipetting tip (1) of the capillary space (23) can be moved in three directions.

3. A process as claimed in claim 2, wherein the reversal of the electric voltage, which causes the sample liquid to exit from the capillary space (23) contained in the supply device, is effected after the pipetting tip (1) comprised by the supply device has reached the position against a detection surface (18).

4. A process as claimed in either of claims 2 and 3, wherein the drive capillary (2) and the pipetting tip (1) can be supplied, by opening a valve (5), with the buffer solution (24), which is stored under pressure in the stock container and has a pH which is suitable for the generation of an electro-osmotic pressure and a suitable ion concentration.

5. A process as claimed in any of claims 1 to 4, wherein electrophoretic deposition of charged biopolymer species on the specimen slide surface (18) is effected by an electroconductive layer on a specimen slide surface (18).

6. An apparatus for the production of biopolymer arrays by micrometering of extremely small amounts of liquid, comprising a supply device for supplying sample substrates to be analyzed, a buffer vessel (14), a drive capillary (2), which connects the supply device and the buffer vessel (14), and comprising connection lines for connecting the drive capillary (2) to a stock container containing a rinsing fluid (24), wherein to apply a voltage between the supply device and the buffer vessel (14) an electrical line (3) is connected to the drive capillary and an electrical contact (4) is connected to the buffer vessel (14) and voltage-reversible switching elements (10) and electrical voltage sources (12a, 12b) are connected to the electrical supply line (3).

7. An apparatus as claimed in claim 6, wherein a flow resistance (13) is incorporated into a branch (16) of the drive capillary (2).

8. An apparatus as claimed in either of claims 6 and 7, wherein the supply device comprises a pipetting tip (1) for the positioning of which in relation to a detection field (18) an X/Y positioning device which effects positioning of the pipetting tip (1) toward the surface of a specimen slide (9) is provided.

9. An apparatus as claimed in any of claims 6 to 8, wherein the drive capillary (2) consists of glass or quartz.

10. An apparatus as claimed in either claim 8 or 9 in conjunction with 8, wherein the pipetting tip (1) is a tip drawn out from a glass capillary to a small diameter, the tip having a diameter in the range from 10 µm to 1000 µm.

11. An apparatus as claimed in claim 10, wherein the pipetting tip (1) is a tip drawn out from a glass capillary to a small diameter with a diameter in the range from 50 µm to 300 µm.

12. An apparatus as claimed in any of claims 8, 9 in conjunction with 8, 10 and 11, wherein an electric earth is provided between the pipetting tip (1) and the drive capillary (2).

13. An apparatus as claimed in any of claims 8 to 12, wherein a platinum wire electrode (3) is provided in a capillary head (21) contained in the supply device.

## Revendications

1. Procédé pour la préparation d'arrangements de biopolymères par microdosage de très petites quantités de liquide, dans lequel on remplit d'abord un dispositif d'amenée, un capillaire d'entraînement (2) reliant le dispositif d'amenée à un récipient de tampon (14) et le récipient de tampon à partir d'un réservoir (11) connectable au capillaire d'entraînement (2) avec une solution de tampon (24), qui permet la production d'un courant électro-osmotique ou éventuellement sert de fluide de balayage, puis par établissement d'une tension électrique sur le capillaire d'entraînement (2) on établit un courant électro-osmotique et on aspire des biopolymères dans le dispositif d'amenée plongé avec une extrémité dans un récipient (8), et enfin après inversion de la tension on dépose des échantillons de biopolymère en quantité dosée sur une surface de détection (18).

2. Procédé selon la revendication 1, **caractérisé par le fait que** le dispositif d'amenée comporte un espace capillaire (23) avec une pointe de pipetage (1), tandis que la pointe de pipetage (1) de l'espace capillaire (23) peut être déplacée dans les trois dimensions.

3. Procédé selon la revendication 2, **caractérisé par le fait que** l'inversion de la tension électrique qui effectue une évacuation du liquide d'échantillon à partir de l'espace capillaire (23) contenu dans le dispositif d'alimentation, est accomplie après qu'a été atteinte la position, par rapport à la surface de détection (18), de la pointe de pipetage (1) que comporte le dispositif d'alimentation.

4. Procédé selon l'une des revendications 2 ou 3, **caractérisé par le fait que** le capillaire d'entraînement (2) et la pointe de pipetage (1) peuvent être alimentés par l'ouverture d'une vanne (5) avec la solution de tampon (24) qui est approvisionnée sous pression dans le réservoir et présente un pH approprié pour la production d'une pression électro-osmotique, ainsi qu'une concentration ionique appropriée.

5. Procédé selon l'une des revendications 1 à 4, **caractérisé par le fait que**, par l'intermédiaire d'une couche conductrice de l'électricité sur une surface de porte-objet (18) une séparation électrophorétique d'espèces biopolymères chargées est réalisée sur la surface de porte-objet (18).

6. Dispositif pour la préparation d'arrangements de biopolymères par microdosage de très petites quantités de liquides ayant un dispositif d'amenée pour l'acheminement de substrats d'échantillons à analyser dans un récipient de tampon (14), un capillaire d'alimentation (2), qui règle l'introduction et relie le récipient de tampon (14), et ayant des conduites de mise en relation pour relier le capillaire d'amenée (2) avec un réservoir contenant un fluide de balayage (24), tandis que pour l'établissement d'une tension entre le dispositif d'amenée et le récipient de tampon (14) sur le capillaire d'amenée, une alimentation électrique (3) et sur le récipient de tampon (14) un contact électrique (4) et sur la conduite électrique (3) des éléments de circuit à polarité inversable (10) et des sources de tension électrique (12a,12b) sont raccordés.

7. Dispositif selon la revendication 6, **caractérisé par le fait que** dans une bifurcation (16) du capillaire d'amenée (2) est disposée une résistance hydraulique (13).

8. Dispositif selon l'une des revendications 6 ou 7, **caractérisé par le fait que** le dispositif d'amenée comporte une pointe de pipetage (1), pour le positionnement de laquelle par rapport à un champ de détection (18) est prévu un dispositif de positionnement en X,Y qui effectue une mise en place de la pointe de pipetage (1) sur la surface d'un porte-objet (9).

9. Dispositif selon l'une des revendications 6 à 8, **caractérisé par le fait que** le capillaire d'amenée (2) est constitué de verre ou de quartz.

10. Dispositif selon l'une des revendications 8 ou 9 en relation avec 8, **caractérisé par le fait que** la pointe de pipetage (1) est une pointe étirée à partir d'un capillaire en verre à un petit diamètre, tandis que la pointe possède un diamètre dans l'intervalle entre 10 µm et 1000 µm.

11. Dispositif selon la revendication 10, **caractérisé par le fait que** la pointe de pipetage (1) est une pointe étirée à partir d'un capillaire en verre à un petit diamètre, avec un diamètre dans l'intervalle entre 50 µm et 300 µm.

12. Dispositif selon l'une des revendications 8, 9 en relation avec 8, 10 et 11, **caractérisé par le fait qu'**une mise à la terre électrique est prévue entre la pointe de pipetage (1) et le capillaire d'amenée (2).

13. Dispositif selon l'une des revendications 8 à 12, **caractérisé par le fait qu'**il est prévu une électrode en fil de platine (3) dans une tête de capillaire (21) contenue dans le dispositif d'amenée.
